# EUROPEAN PATENT APPLICATION

(11) **EP 4 059 422 A1**
(43) Date of publication of application: **21.09.2022**
(21) Application number: 21163785.5
(22) Date of filing: 19.03.2021
(51) Int. Cl.: A61B 5/12, G01H 15/00

(54) **SYSTEM FOR DETECTING MIDDLE OR INNER EAR CONDITIONS**

(71) Applicant: Koninklijke Philips N.V., 5656 AG Eindhoven (NL)
(72) Inventor: WRIGHT, Christopher John, 5656 AE Eindhoven (NL)
(74) Representative: Philips Intellectual Property & Standards

(57) **Abstract**

The invention provides a system for detecting middle or inner ear conditions, based on the analysis of otoacoustic emissions, thereby to distinguish between a normal ear and an ear condition to be detected.

## Description

### FIELD OF THE INVENTION

The invention relates to a system for detecting middle or inner ear conditions..

### BACKGROUND OF THE INVENTION

Diagnosis of ear conditions is typically performed by a GP based on a visual inspection. It is also known to perform hearing tests by measuring otoacoustic reflections in response to introduced sounds, using a tympanometer.

Wax build-up in the outer ear often prevents GPs from being able to diagnose underlying conditions which may need attention, such as otitis media or inflammation. This wax also prevents standard methods of producing and receiving otoacoustic emissions.

Wax removal procedures may require specialists and can significantly delay the time to diagnosis. In addition to wax, visual inspection techniques may sometimes be difficult due to other reasons, such as unusual ear canal geometries or with children or other patients who move or who are uncooperative.

There is there for a need for a system which can detect conditions in a more user friendly way, and for example even in the presence of wax.

### SUMMARY OF THE INVENTION

The invention is defined by the claims.

According to examples in accordance with an aspect of the invention, there is provided a system for detecting middle or inner ear conditions, comprising:
an input for receiving an otoacoustic emission signal generated by a measurement device in response to eye movements; and
a controller for analyzing the otoacoustic emission signal thereby to distinguish between at least a first type of otoacoustic emission signal indicative of a normal ear and a second type of otoacoustic emission signal indicative of an ear condition to be detected.

This system processes an otoacoustic emission signal in order to distinguish between a normal ear and an ear with an ear condition to be detected. The system may be designed for a particular ear condition such as otitis media or a perforated eardrum, or else it may be designed to distinguish between multiple conditions. For example, a perforated eardrum will introduce a broader frequency response. Increased tension of the eardrum e.g. due to inflammation, will cause a frequency shift. Different ear conditions and also wax build-up will also result in different sound damping effects, which will influence both the temporal and spectral (frequency) characteristics.

Thus, the invention is based on the recognition that pathologies of the eardrum or middle ear such as otitis media may naturally result in changes to the emitted sounds due to fluid presence, membrane perforation, cholesteatoma, or other conditions which may affect the eardrum oscillations in characteristic ways.

For example, a cholesteatoma is an example of a rare but critical pathology of the eardrum which will result in a change to the way that the membrane oscillates (and therefore the acoustic emission).

The invention provides a non-invasive way to detect one or more ear conditions, which can also be effective even in the presence of wax, which may render ineffective standard acoustic analysis using a tympanometer. The invention thus avoids the need for wax removal procedures, and these may require specialists and can significantly delay the time to diagnosis.

In addition to wax, visual inspection techniques may sometimes be difficult due to other reasons, such as unusual ear canal geometries or with children or other patients who move or who are uncooperative. The invention thus addresses these issues.

The system also provides diagnostic value just in detecting a healthy ear condition, which would aid a GP to determine if further testing is required or not.

The system may further comprise an output device for providing an output instruction to a user of the system to perform particular eye movements. In a most basic implementation, the eye movements which result in the otoacoustic emission signal may be a standard set of movements, and the user then learns how to use the system. There may for example be an output instruction which is simply to perform a pre-learned set of movements. Alternatively, the user may be prompted by an assisting medical professional to perform particular eye movements.

However, in a preferred example, the system has an output device for guiding the user to perform the required eye movements for which the system is designed (i.e. the signal analysis that is conducted). This provides an easy to use system for the user.

The output instruction may also be dynamically adapted based on the analysis of the otoacoustic emission signal.

The controller may be adapted to select an output instruction in dependence on the ear condition to be detected. The particular eye movements to be performed may in this way be tailored to a particular condition or conditions to be detected. Furthermore, a sequence of different eye movements may be used to enable more reliable detection, or to enable location of a particular condition such as the location of an eardrum perforation.

The output device may comprise a display device for directing the gaze of the user in a particular direction. A display provides a convenient way to deliver eye movement instructions to a user, by instructing them to following the movement of a displayed icon with their gaze.

The controller may be adapted to apply filtering to the otoacoustic emission signal to extract signal components associated with the eye movements. In this way, the features of the otoacoustic emission signal which derive from the eye movements are extracted.

The controller may be adapted to apply temporal and/or spectral filtering. The temporal filtering for example aligns the signal to be analyzed with the time during which eye movements are made. Temporal filtering may also obtain signal damping information, i.e. time decay information. Spectral filtering obtains frequency characteristics, which will also depend on the condition to be detected.

The controller may be adapted to select filtering characteristics in dependence on the eye movements. Different eye movements (e.g. amplitude and speed) will result in different otoacoustic signal characteristics, so different filtering characteristics may be needed to obtain the relevant signal features.

The system may further comprise a database storing reference characteristics, and wherein the controller is adapted to obtain otoacoustic emission signal characteristics from the extracted signal components and compare the obtained otoacoustic emission characteristics with the reference characteristics, thereby to distinguish between said at least first and second types of otoacoustic emission signal. The reference characteristics are for example based on previously collected data from a general population.

The controller may be adapted to compare by applying a pattern matching function in the temporal and/or frequency domains. A standard error in the frequency domain and/or amplitude domain may be calculated as the result of the pattern matching. Temporal matching may involve finding an average similarity at multiple different time differences between the reference characteristics and the extracted signal components. A most similar (hence lowest standard error) match value may be used as the result of the pattern matching. More advanced matching algorithms may also be used, such as dynamic time warping.

Recent advances in machine learning analysis of subtle acoustic signatures (e.g. automatically detecting different valvular heart conditions types, speech recognition advances, etc.), mean that different conditions may be reliably differentiated using automated, or AI assisted, acoustic analysis.

The database may further store historical otoacoustic emission signal characteristics for the user of the system. In this way, in addition to using general reference characteristics, changes may be observed for the particular user over time.

The system may further comprise the measurement device for generating the otoacoustic emission signal, wherein the measurement device comprises a microphone. This is a non-invasive way to generate the otoacoustic emission signal. High sensitivity and low cost of microphones has been driven by advances in MEMS microphone technology.

The microphone may be fitted to an ear bud or fitted to a rim of an ear entry projection of an otoscope. The microphone may be the microphone of a tympanometer.

Thus, the microphone may be implemented in different ways, including use of the existing microphone of a tympanometer (but analyzing acoustic signal emissions rather than acoustic signal reflections).

The invention also provides a computer-implemented method for detecting a likely presence of a middle or inner ear condition, comprising:
receiving an otoacoustic emission signal generated by a measurement device; and
analyzing the otoacoustic emission signal thereby to distinguish between at least a first type of otoacoustic emission signal indicative of a normal ear and a second type of otoacoustic emission signal indicative of an ear condition to be detected.

The invention also provides a computer program comprising computer program code which is adapted, when said program is run on a computer, to implement the method.

These and other aspects of the invention will be apparent from and elucidated with reference to the embodiments) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a system for detecting middle or inner ear conditions; and
Figure 2 shows an acoustic analysis method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for detecting middle or inner ear conditions, based on the analysis of otoacoustic emissions, thereby to distinguish between a normal ear and an ear condition to be detected.

The invention is based on the use of spontaneously emitted otoacoustic emission, produced in the tympanic membrane, due to eye movements. These emissions can for example be measured by a microphone placed in the outer ear canal.

Otoacoustic emissions produced by eye movements are louder than standard actively stimulated response emissions (for example 57 dB SPL compared to 0- to 20 dB SPL), and are in a lower frequency range (∼30Hz).

These emissions are also reliable, and vary with the direction and amplitude of eye moment. For example, it has been reported that when the eyes move left, both eardrums initially move right and then oscillate for three to four cycles, with another one to two cycles occurring after the eye movement is complete. Eye movements in the opposite direction produce an oscillatory pattern in the opposite direction.

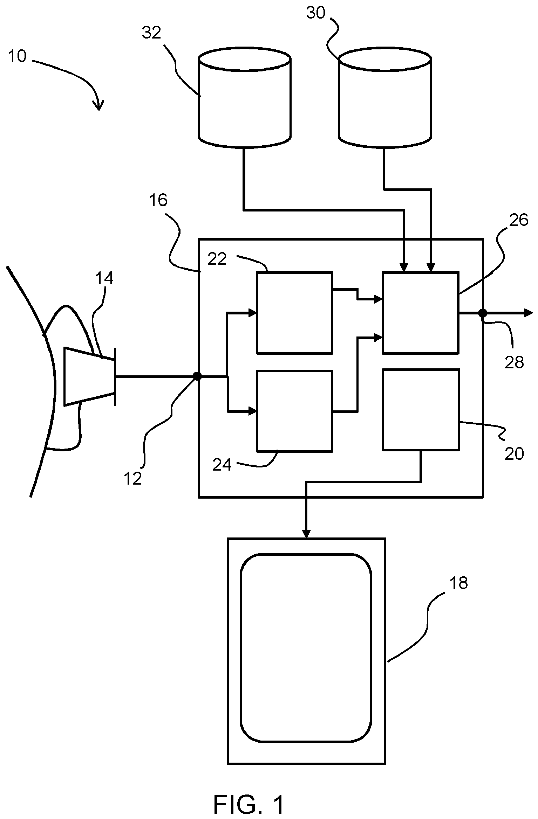

Figure 1 shows a system 10 for detecting middle or inner ear conditions. The system comprises an 12 input for receiving an otoacoustic emission signal generated by a measurement device 14 in response to eye movements.

The measurement device 14 comprises a microphone which may be fitted to an ear bud (which may be located in the outer ear or inserted into the ear canal) or fitted to a rim of an ear entry projection of an otoscope. It may be the microphone of a tympanometer.

The microphone 14 collects acoustic data comprising amplitude and frequency parameters.

A controller 16 is used to analyze the otoacoustic emission signal thereby to distinguish between at least a first type of otoacoustic emission signal indicative of a normal ear and a second type of otoacoustic emission signal indicative of an ear condition to be detected.

In the example shown, an output device 18 provides an output instruction to a user of the system to perform particular eye movements. The output device is a display device for directing the gaze of the user in a particular direction.

The controller may select an output instruction, i.e. a gaze following instruction, in dependence on the ear condition to be detected. For this purpose, the controller has a gaze direction algorithm 20 which selects desired eye movements and instructs the output device 18 accordingly. In the case of a display device, the instructions to the display device are pixel display commands.

The gaze direction algorithm selects or designs gaze directions to deliver to the user. In a simplest embodiment, this may consist of a lookup table with visual display settings in terms of pixel color intensity, coordinates and timing which are associated with the condition to be investigated.

The pixel display commands for example result in a displayed feature on the output device which directs the patient's gaze in a defined pattern with controlled parameters. The displayed feature may produce eye movements which perform a specific test of the tympanic membrane condition.

Parameters defined by the pixel display commands may include:
the speed of movement of a feature (and therefore of the patient's eyes);
the amplitude of the feature movement;
the direction of the feature movement;
the timing of the feature movement.

The pixel display commands which are ideal for each condition may be determined prior to implementation of the system through a testing phase.

In order for the movement of the feature to correspond to a known movement of the eyes, the position of the user relative to the display needs to be known, so that the speed amplitude and direction of eye movements remain consistent. This may be achieved by instructions on the screen, instructing the patient to occupy an approximate position relative to the screen. Optionally, the position of the patient may be sensed using a camera, and instructions may be provided to instruct the patient to move when not in a suitable position.

Note that instead of using a display, the gaze cues may be given by a medical professional during a visual aural exam, such as an active tympanometry exam. Information may be derived from both conventional reflected otoacoustic emissions as well as from emissions induced by eye gaze movement.

The controller 16 processes the otoacoustic signal using a temporal filter 22 and a spectral filter 24. The filtering characteristics for example depend on the eye movements. Thus, the filter parameters may depend on the chosen pixel display commands and hence on the condition to be tested.

A diagnosis algorithm 26 processes the filtered information to provide an output 28 to the user which indicates if the ear is normal of if the condition being investigated is present.

In order to make the diagnosis, a signature database 30 stores reference characteristics. These comprise acoustic data patterns which are known to be associated with healthy or pathogenic states of the ear. These patterns may be acoustic data sequences which are linked to good or bad health states in prior testing, such as a frequency variation over time which may be associated with otitis media or other conditions. Optionally, relative threshold measures may be used, such as 10% lower emission frequency than a past recorded signature, or a signature recorded from the other ear.

In all cases, the obtained otoacoustic emission signal characteristics are obtained from the extracted signal components (generated by the filtering), and they are compared with the reference characteristics, thereby to distinguish between different types of otoacoustic emission signal. This comparison may be made by applying a pattern matching function in the temporal and/or frequency domain.

A patient database 32 is also provided with historical otoacoustic emission signal characteristics for the user of the system. This stores current emission data for retrieval in later sessions.

The system may be designed for a particular ear condition such as otitis media or a perforated eardrum, or else it may be designed to distinguish between multiple conditions.

An input to the system may allow a user to define the desired condition to be investigated, such as a pathology type, or tympanic property to investigate. For example, a perforated eardrum will introduce a broader frequency response. Increased tension of the eardrum e.g. due to inflammation, will cause a frequency shift. Different ear conditions and also wax build-up will also result in different sound damping effects, which will influence both the temporal and spectral (frequency) characteristics.

Thus, the invention is based on the recognition that pathologies of the eardrum or middle ear such as otitis media may naturally result in changes to the emitted sounds due to fluid presence, membrane perforation, cholesteatoma, or other conditions which may affect the eardrum oscillations in characteristic ways.

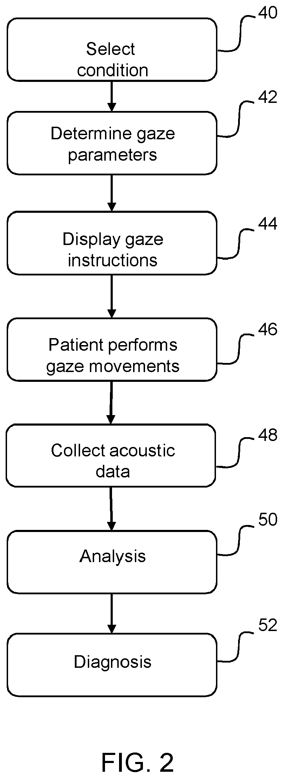

Figure 2 shows a computer-implemented method for detecting middle or inner ear conditions.

In step 40, a condition to be tested is selected. This may be based on an input by a GP to the system, or else the system may itself determine a suitable condition to be tested, based on an initial default test.

In step 42, the suitable gaze parameters are determined, which, when a display device is used, will comprise pixel display commands.

For example, if the system is to determine a suitable condition to test itself, and hence determine the most suitable gaze direction instructions to prepare, a standard generic pixel display command may be selected by default. This will acquire a range of diagnostic information. Subsequently, the system may use existing diagnostic information to query a lookup table of conditions, and select one to be tested. This process may repeat for multiple iterations.

In step 44 corresponding gaze commands are sent to the display device, thereby providing visual gaze instructions to the patient.

In step 46, the patient follows the gaze directing instructions.

In step 48, the otoacoustic emissions are collected by the sensor.

In step 50, the acoustic data are analyzed. As explained above, this may involve spectral and/or temporal filtering, and the filter parameters are set in dependence on the gaze instructions.

For example, the otoacoustic emissions are known to occur simultaneously or slightly preceding the actual eye movement. Therefore, the timestamp of a pixel display command (minus a small time interval (e.g. 100ms)) may be used to temporally filter the acoustic data. This defines a time sequence of acoustic data that relates to a particular pixel display command.

The spectral filtering is for example used to exclude noise.

In step 52 a determination is made. Preferably, this not a full diagnosis, but an indication of whether further investigations are warranted. This involves querying the signature database 30 to retrieve the reference characteristics (which comprises signatures for different ear states). Information of diagnostic relevance is then determined by measuring the similarity of the acoustic data to the reference characteristics:
A standard pattern matching process may be employed where the standard error of the data points in the frequency domain and/or amplitude domain may be calculated. Some degree of temporal matching may be performed where the average similarity is calculated at multiple different time differences between the acoustic data and reference characteristics. The most similar (lowest standard error) match value may be used from these. More advanced matching algorithms may also be used, such as dynamic time warping.

If the error score is below a pre-defined threshold, a successful match may be identified.

The ear state may be monitored by making a comparison with previous data, from the patient database 32. In this case, past patient acoustic data is retrieved from the patient database and compared with the current acoustic data, or acoustic data may be compared between the two ears.

A change in frequency or amplitude data points between the two sources of data may be calculated for the time-aligned acoustic data. An average frequency or amplitude change may be determined for the whole sequence. The change measurement may then be assessed using the reference characteristics. If the change is above a threshold, a successful match may be identified.

For all successful matches, the relevant diagnostic information is retrieved and output from the signature database 30. This information is output and also stored in the patient database.

As mentioned above, the use of a display is optional. A lower cost system may rely on gaze direction instructions given by the GP. The GP may be trained to instruct patients to follow an approximate gaze pattern.

For example, with an earbud microphone in place, a patient may be asked to focus on a finger on one hand and then move their gaze to a finger on another hand. The GP's hands may therefore be moved to approximate positions relative to the patient's gaze field which allow a general or targeted test to be performed.

Instead of, or as well as, following a feature of a display, gaze direction instructions may also be delivered acoustically using a speaker. These may comprise verbal instructions, for example requesting the patient to look in approximate directions, such as 'Look to the center of your visual field', 'Now look left and up' etc.

Acoustic instructions may also comprise make use of virtual sound source direction. A virtual sound location may be produced in known manner using e.g. phase modulation, at a desired location in the patient's perceptual field. Multiple such sounds may be produced and the patient may be requested to look visually to the produced locations.

The invention provides a way of eliciting controlled otoacoustic emissions from a patient which may be used to detect medically relevant information relating to the condition of the tympanic membrane and related ear structures.

The system provides the ability to perform a sensitive diagnostic test which differentiates between causes of hearing loss or other symptoms, with low-cost equipment (only an ear-located microphone and processing algorithms in a simplest embodiment).

The problem of ear-wax is reduced compared to otoscopy and standard active tympanometry due to the collection of low frequency and high amplitude otoacoustic signals (relative to active tympanometry). There is only a single pass rather than a double pass through the ear canal because the tympanic membrane is used as a sound source rather than a reflector. The diagnostic value of the collected data is optimized by designing the most suitable gaze directions given to the user.

Some possible implementations will now be discussed in more detail.

In one example, a generic test is performed, and this is sufficient to differentiate between ear conditions. The most likely condition is otitis media, as this is one of the most common middle/outer ear conditions. A suitable test may consist of moving the eyes horizontally between two points on a screen, left to right, and then right to left, which generates a strong recordable oscillation in the eardrums of the opposite phase in each ear.

More extensively, a test may given where the user moves their gaze to a neutral position (directly in front of the user's head at eye level), and then follows a point which is horizontally to the left. A series of similar tests may be performed by always returning to the central neutral gaze location before following points on the circumference of a circle, progressing at 45 degree (or suitable) increments. This will cause a range of oscillation profiles (which are different for different eye movement angles) in the eardrum which are predictable and are differently affected by the different eardrum conditions such as perforation vs otitis media.

Different angles of eye movement have differential signal to noise ratios for detecting certain conditions, or the spatial properties of those conditions, due to the different spatial and frequency properties of mechanical oscillations created in the eardrum and surrounding tissues, and how these are affected by the mechanical tissue changes induced by otitis media, perforation etc..

Another example is based on previous analysis of the exact spatial nature of the oscillations within the ears for different conditions. Using this analysis, targeted tests may be delivered to diagnose specific conditions quickly and at higher resolution. In such cases, the user is directed to perform a gaze task involving certain saccade angles which result in a high signal to noise ratio for detection and characterization of the specific condition.

In the case of otitis media, where the middle ear is filled with fluid, this condition has a severe damping effect on the oscillations, reducing the frequency, amplitude and total number of measurable cycles.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system (10) for detecting middle or inner ear conditions, comprising:
an input (12) for receiving an otoacoustic emission signal generated by a measurement device in response to eye movements; and
a controller (16) for analyzing the otoacoustic emission signal thereby to distinguish between at least a first type of otoacoustic emission signal indicative of a normal ear and a second type of otoacoustic emission signal indicative of an ear condition to be detected.

2. The system of claim 1, further comprising an output device (18) for providing an output instruction to a user of the system to perform particular eye movements.

3. The system of claim 2, wherein the controller (16) is adapted to select an output instruction in dependence on the ear condition to be detected.

4. The system of claim 2 or 3, wherein the output device (18) comprises a display device for directing the gaze of the user in a particular direction.

5. The system of any one of claims 1 to 4, wherein the controller (16) is adapted to apply filtering to the otoacoustic emission signal to extract signal components associated with the eye movements.

6. The system of claim 5, wherein the controller (16) is adapted to apply temporal and/or spectral filtering.

7. The system of claim 5 or 6, wherein the controller (16) is adapted to select filtering characteristics in dependence on the eye movements.

8. The system of any one of claims 5 to 7, further comprising a database (30,32) storing reference characteristics, and wherein the controller is adapted to obtain otoacoustic emission signal characteristics from the extracted signal components and compare the obtained otoacoustic emission characteristics with the reference characteristics, thereby to distinguish between said at least first and second types of otoacoustic emission signal.

9. The system of claim 8, wherein the controller (16) is adapted to compare by applying a pattern matching function in the temporal and/or frequency domain.

10. The system of claim 8 or 9, wherein the database (30,32) further stores historical otoacoustic emission signal characteristics for the user of the system.

11. The system of any one of claims 1 to 10, further comprising the measurement device (14) for generating the otoacoustic emission signal, wherein the measurement device comprises a microphone.

12. The system of claim 11, wherein the microphone (14) is fitted to an ear bud or fitted to a rim of an ear entry projection of an otoscope.

13. The system of claim 11, wherein the microphone (14) is the microphone of a tympanometer.

14. A computer-implemented method for detecting a likely presence of a middle or inner ear condition, comprising:
(48) receiving an otoacoustic emission signal generated by a measurement device; and
(50) analyzing the otoacoustic emission signal thereby to distinguish between at least a first type of otoacoustic emission signal indicative of a normal ear and a second type of otoacoustic emission signal indicative of an ear condition to be detected.

15. A computer program comprising computer program code which is adapted, when said program is run on a computer, to implement the method of claim 14.
